# EUROPEAN PATENT SPECIFICATION

(11) **EP 3 560 495 B1**
(45) Date of publication and mention of the grant of the patent: **28.07.2021**
(21) Application number: 17888272.6
(22) Date of filing: 25.12.2017
(51) Int. Cl.: A61K 31/4174, A61L 31/08, A61M 37/00, A61P 25/20, A61K 9/00, A61K 47/18, A61K 47/20

(54) **MICRONEEDLE DEVICE**
MIKRONADELVORRICHTUNG
DISPOSITIF À MICRO-AIGUILLE

(30) Priority: 26.12.2016 JP 2016250998
(43) Date of publication of application: 30.10.2019
(73) Proprietor: Hisamitsu Pharmaceutical Co., Inc., Tosu-shi, Saga 841-0017 (JP)
(72) Inventor: HORI, Ryota, Tsukuba-shi Ibaraki 305-0856 (JP); KOGURE, Toshihiro, Tsukuba-shi Ibaraki 305-0856 (JP); TOKUMOTO, Seiji, Tsukuba-shi Ibaraki 305-0856 (JP); NISHIMURA, Shinpei, Tsukuba-shi Ibaraki 305-0856 (JP)
(74) Representative: Grünecker Patent- und Rechtsanwälte PartG mbB
(86) International application number: PCT/JP2017/046456
(87) International publication number: WO 2018/123982

(56) References cited:
- WO-A1-2016/039418
- WO-A2-2010/063030
- JP-A- H05 503 916
- JP-A- 2001 506 904
- JP-A- 2014 507 473
- JP-A- 2014 508 746
- JP-A- 2016 532 642
- KIVISTO K T ET AL: "Pharmacokinetics and pharmacodynamics of transdermal dexmedetomidine", EUROPEAN JOURNAL OF CLINICAL PHARMACOLOGY, SPRINGER VERLAG, DE, vol. 46, no. 4, 1 January 1994 (1994-01-01), pages 345-349, XP008124147, ISSN: 0031-6970

## Description

### Technical Field

The present invention relates to a microneedle device.

### Background Art

Dexmedetomidine acts on α₂ adrenergic receptors so as to exhibit a sedative effect. Dexmedetomidine is used, for example, for sedation during artificial respiration and after removal of the artificial respiration in an intensive care, or for sedation during non-intubated surgery or treatment under local anesthesia. For such applications, dexmedetomidine is provided in a form of hydrochloride as liquid medicine for intravenous injection.

On the other hand, transdermal administration using a microneedle device is known as a form of administering an agent (for example, Patent Literature 1). A microneedle device allows microneedles to make punctures in a stratum corneum layer as the outermost layer of the skin so as to form fine holes through which an agent passes, so that the agent can be transdermally administered. The transdermal administration of dexmedetomidine using a microneedle device has not been conventionally studied. Kivistö et al disclose the pharmacokinetics and pharmacodynamics of transdermal dexmedetomidine (Non-Patent Literature 1).

### Citation List

### Patent Literature

Patent Literature 1: Japanese Unexamined Patent Publication No. 2001-506904 Non-Patent Literature 1: Kivistö et al, Eur J Clin Pharmacol (1994) 46: 345-349

### Summary of Invention

### Technical Problem

The present inventors have attempted administration of dexmedetomidine hydrochloride using a microneedle device to find that the increase rate of dexmedetomidine concentration in plasma is extremely slower than in the case of intravenous injection. A slow increase of dexmedetomidine concentration in plasma makes it difficult to obtain an expected therapeutic effect at an expected time.

### Solution to Problem

Through intensive study, the present inventors have found that blending isoproterenol with dexmedetomidine hydrochloride improves the increase rate of dexmedetomidine concentration in plasma, and have thereby accomplished the present invention.

The present invention is a microneedle device comprising a substrate, microneedles disposed on the substrate, and a coating formed on the microneedles, wherein the coating comprises dexmedetomidine or a pharmaceutically acceptable salt thereof and isoproterenol or a pharmaceutically acceptable salt thereof.

A total amount of isoproterenol and a pharmaceutically acceptable salt thereof may be 0.3 parts by mass or more based on 100 parts by mass of a total amount of dexmedetomidine and a pharmaceutically acceptable salt thereof in the coating.

The coating may further comprise a stabilizer, and the stabilizer may be L-cysteine or sodium pyrosulfite.

### Advantageous Effects of Invention

According to the microneedle device of the present invention, a fast increase rate of dexmedetomidine concentration in plasma after administration of dexmedetomidine or a pharmaceutically acceptable salt thereof is achieved.

### Brief Description of Drawings

Figure 1 is a perspective view showing a microneedle device in an embodiment.
Figure 2 is a cross-sectional view taken from line II-II of Figure 1.
Figure 3 is a graph showing the change with time in the dexmedetomidine (DEX) concentration in plasma in Test Example 1.
Figure 4 is a graph showing the change with time in the dexmedetomidine (DEX) concentration in plasma in Test Example 2.
Figure 5 is a graph showing the change with time in the dexmedetomidine (DEX) concentration in plasma in Test Example 3.

### Description of Embodiments

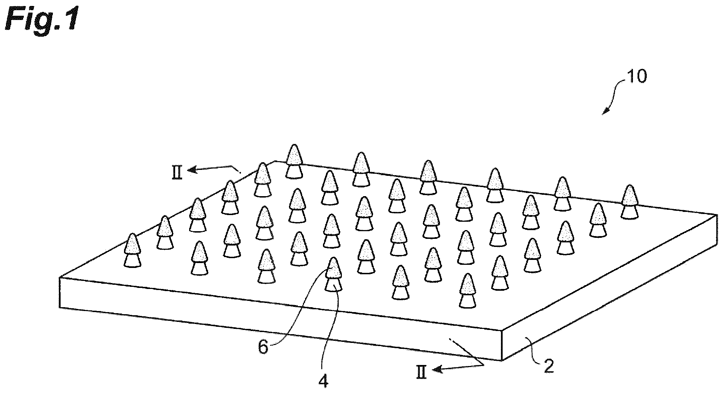

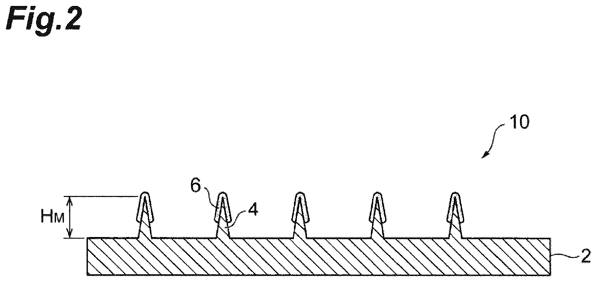

The microneedle device of the present invention comprises a substrate, microneedles disposed on the substrate, and a coating formed on the microneedles. The microneedle device of the present invention in an embodiment is shown in Figure 1 and Figure 2. A microneedle device 10 comprises a substrate 2, a plurality of microneedles 4 disposed on the surface of the substrate 2, and a coating 6 formed on the microneedles 4. In the present specification, a structure having microneedles 4 formed on the substrate 2 is referred to as a microneedle array. As the microneedle array, a conventionally known microneedle array may be used. An example of the detail of the microneedle array is as follows.

The substrate 2 is a foundation for supporting the microneedles 4. The shape and the form of the substrate are not particularly limited, and are, for example, a rectangular shape or a circular shape and a flat form or a curved form. The area of the substrate 2 is, for example, 0.5 cm² to 10 cm², or 1 cm² to 3 cm².

The microneedle 4 is a convex structure, which denotes a needle shape in a broad sense or a structure comprising a needle shape. The microneedle 4 is not limited to a structure having a needle shape with a pointed end, and may be in a shape without a pointed end. The microneedle 4 is, for example, in a polygonal pyramid shape such as a quadrangular pyramid shape or a conical shape. The microneedle 4 is a microstructure having a length (height) of, for example, 300 µm to 500 µm.

The microneedles 4 are arranged, for example, in a square lattice form, a rectangular lattice form, an orthorhombic lattice form, a 45-degree staggered form, or a 60-degree staggered form. From the perspective of efficiently introducing dexmedetomidine or a pharmaceutically acceptable salt thereof in the coating 6 into the skin, the number of the microneedles 4 per 1 cm² of a substrate 2 may be 100 to 10000, and the number is preferably 200 to 5000, more preferably 400 to 850.

Examples of the material of the substrate 2 or the microneedle 4 include silicon, silicon dioxide, ceramics, metals, polysaccharides, and synthesized or natural resin materials. More specifically, synthesized or natural resin materials including a biodegradable polymer such as polylactic acid, polyglycolide, polylactic acid-co-polyglycolide, pullulan, caprolactone, polyurethane and polyanhydride, or a non-degradable polymer such as polycarbonate, polymethacrylate, ethylene vinyl acetate, polytetrafluoroethylene and polyoxymethylene are preferred.

The coating 6 may be formed on all of the plurality of microneedles 4 that exist, or may be formed on a part of the microneedles 4 only. The coating 6 may be formed on a tip part only of the microneedle 4, or may be formed to cover the whole of the microneedle 4. The average thickness of the coating 6 may be less than 50 µm, or may be 1 µm to 30 µm.

The coating comprises dexmedetomidine or a pharmaceutically acceptable salt thereof (e.g., hydrochloride) and isoproterenol or a pharmaceutically acceptable salt thereof (e.g., hydrochloride). Hereinafter, "dexmedetomidine or a pharmaceutically acceptable salt thereof' may be referred to as "dexmedetomidine", and "isoproterenol or a pharmaceutically acceptable salt thereof" may be referred to as "isoproterenol" in some cases. These terms are used as having the same meaning unless a particular distinction is made.

The amount of coating per 1 cm² of a substrate may be 10 µg to 400 µg, or 20 µg to 300 µg. Although depending on the purpose of treatment, the total content of dexmedetomidine and a pharmaceutically acceptable salt thereof in the total amount of coating may be 10 mass% to 90 mass%, and is preferably 40 mass% to 85 mass%, more preferably 60 mass% to 80 mass%, in order to obtain substantial therapeutic effect of dexmedetomidine.

From the perspective of improving the increase rate of dexmedetomidine concentration in plasma, the total amount of isoproterenol and a pharmaceutically acceptable salt thereof based on 100 parts by mass of a total amount of dexmedetomidine and a pharmaceutically acceptable salt thereof in the coating may be 0.3 parts by mass or more, and preferably 1.1 parts by mass or more, more preferably 3.4 parts by mass or more, particularly preferably 6.9 parts by mass or more. The total amount of isoproterenol and a pharmaceutically acceptable salt thereof in the total amount of coating may therefore be, for example, 0.2 mass% or more, 0.8 mass% or more, 2.4 mass% or more, or 4.8 mass% or more. From the perspective of inhibiting side effects of isoproterenol (palpitations and paresthesia of face or scalp), it is preferable that the total amount of isoproterenol and a pharmaceutically acceptable salt thereof in the coating be 1 µg or less. The total amount of isoproterenol and a pharmaceutically acceptable salt thereof may be, for example, 12 mass% or less, 9.5 mass% or less, 9.0 mass% or less, 5.5 mass% or less, 5.0 mass% or less, or 4.8 mass% or less, based on the total amount of coating, and may be 18 parts by mass or less, 8.5 parts by mass or less, 8.0 parts by mass or less, or 7.0 parts by mass or less, based on 100 parts by mass of a total amount of dexmedetomidine and a pharmaceutically acceptable salt thereof.

The coating may further comprise biologically inactive components. The total amount of the biologically inactive components in the total amount of coating is, for example, 0 mass% to 70 mass%. Examples of the biologically inactive components include a base, a stabilizer, a pH adjuster, and other components (e.g., components for accelerating transfer of drugs into blood, a surfactant, oils and fats, or inorganic substances). Also, the coating may further comprise components known as vasodilator such as nicotinic acid amide, in addition to isoproterenol.

The base performs function of retaining a drug to the microneedles, and also increases the viscosity of a coating composition used for forming the coating so as to exhibit an effect of easier application to the microneedle. Examples of the base include water-soluble polymers such as polysaccharides, cellulose derivatives, biodegradable polyester, biodegradable polyamino acid, polyethylene oxide, polyvinyl alcohol, and polyvinylpyrrolidone, and low-molecular weight molecules such as sugar, sugar alcohol and ascorbic acid.

Examples of the stabilizer include L-cysteine, sodium pyrosulfite, sodium hydrogen sulfite, ascorbic acid, ehtylenedimamine tetraacetic acid (EDTA) or salts thereof, and dibutylhydroxytoluene (BHT). Among these, L-cysteine, sodium pyrosulfite and sodium hydrogen sulfite are more preferred from the perspective of stabilizing dexmedetomidine and isoproterenol. These stabilizers may be used alone, or multiple stabilizers may be used in combination.

As the pH adjuster, an inorganic acid or an organic acid, an alkali, a salt, an amino acid or a combination thereof, which is typically used as a pH adjuster for agents, may be used.

Next, a method for producing a microneedle device will be described. A microneedle device is produced by applying a coating composition comprising dexmedetomidine and isoproterenol to microneedles of a microneedle array, and drying the composition to form a coating on the microneedles. Coating may be performed, for example, by filling a reservoir having multiple hollows with the coating composition, and dipping the microneedles therein.

The coating composition may comprise a solvent (water, polyhydric alcohols, lower alcohols, triacetin, etc.) for dissolving dexmedetomidine, isoproterenol and other components that form a coating. All or a part of the solvent is removed in the step of drying the coating composition.

### Examples

### (Preparation of microneedle device)

In the following examples, microneedle devices were prepared by the following method.

Each of the components that form a coating and a suitable amount of purified water (and ethanol, if required) were mixed to produce a solution of a coating composition. Subsequently, the tips of the microneedles were dipped in the solution and the solution was air-dried to form a coating on the microneedles. Dipping was performed such that the amount of dexmedetomidine hydrochloride in the coating was controlled at a level of about 30 to 60 µg.

### (Analysis of increase rate of dexmedetomidine concentration)

In the following examples, the increase rate of dexmedetomidine (Dex) concentration in plasma was analyzed as follows.
1) A microneedle device was applied to the skin with hair removed in the ventral region of a male dog, and blood was collected after 5, 10, 20, 30, 60 and 90 minutes.
2) The dexmedetomidine concentration in plasma was obtained by quantifying the dexmedetomidine in plasma by liquid chromatography-mass spectrometry (LC-MS).
3) From the change with time in the value obtained by dividing the dexmedetomidine concentration by the dosage of dexmedetomidine, the increase rate of dexmedetomidine concentration was analyzed. The dosage of dexmedetomidine was calculated from the amount of dexmedetomidine hydrochloride blended in the coating.

### (Measurement of amount of each component transferred into skin)

In the following examples, each of the components in the coating which had been transferred into the skin was obtained as follows.
1) After a microneedle device was applied for 10 seconds, each of the components remaining on the skin surface and on the microneedle device (e.g., dexmedetomidine hydrochloride or a vasodilator) was collected, and each amount (residual amount) was measured by HPLC method.
2) Separately, another microneedle device having the same features was prepared to measure the amount of each of the components in the coating (initial amount).
3) From the initial amount and the residual amount thus obtained, the amount of each of the components which had been transferred into the skin was calculated.

### (Analysis of storage stability)

In the following examples, the storage stability of dexmedetomidine hydrochloride or isoproterenol hydrochloride in the coating was determined as follows.
1) A microneedle device after preparation was seal-packed with an aluminum laminate packaging material.
2) After 3 days from preparation of the microneedle device, the amount (initial amount) of dexmedetomidine hydrochloride or isoproterenol hydrochloride in the coating was measured by HPLC method.
3) Separately, a microneedle seal-packed in an aluminum laminate packaging material in the same manner was prepared, and after storage at 50°C for one week, the amount of dexmedetomidine hydrochloride or isoproterenol hydrochloride in the coating was measured by HPLC method.

### (Test Example 1)

A microneedle device having a coating comprising components shown in Table 1 was prepared. "Base" in the table is a biologically inactive component comprising a water-soluble polymer (the same shall apply hereinafter). The specification of the microneedle array used is as follows.
Material: polylactic acid
Shape of microneedle: quadrangular pyramid
Length of microneedle: 500 µm
Arrangement of microneedles: lattice form
Number of microneedles: 640
Area of the substrate where microneedles are arranged: about 1 cm²

**[Table 1]**

| Component | Comparative Example 1 |
|---|---|
| Dexmedetomidine hydrochloride | 68 |
| Base | 18 |
| L-cysteine | 7.5 |
| Total (part by mass) | 93.5 |

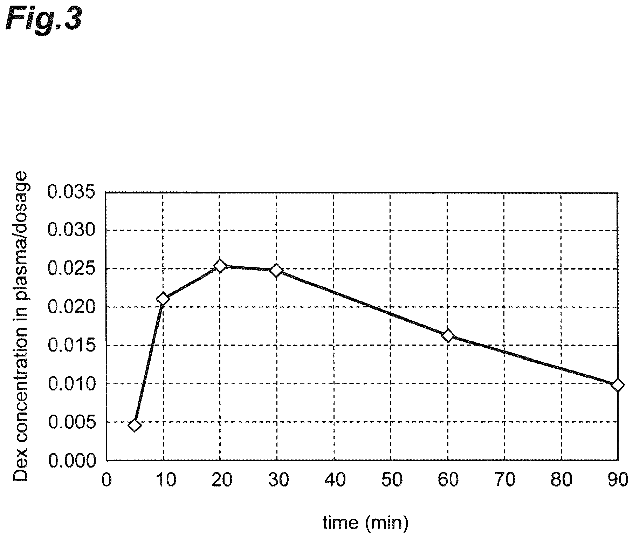

A microneedle was applied to a male dog, and the increase rate of dexmedetomidine concentration in plasma was analyzed by the method described above. The results are shown in Figure 3. When a microneedle device comprising no isoproterenol hydrochloride was applied, it took time until the dexmedetomidine concentration in plasma increased. The amount of dexmedetomidine hydrochloride transferred into the skin was 58.5 µg in 90 minutes.

### (Test Example 2)

Microneedle devices having a coating comprising components shown in Table 2 were prepared. The specification of the microneedle array used is as follows.
Material: polylactic acid
Shape of microneedle: quadrangular pyramid
Length of microneedle: 500 µm
Arrangement of microneedles: lattice form
Number of microneedles: 156
Area of the substrate where microneedles are arranged: about 1 cm².

**[Table 2]**

| Component | | Example 1 | Comparative Example 2 | Comparative Example 3 | Comparative Example 4 |
|---|---|---|---|---|---|
| Dexmedetomidine hydrochloride | | 36.5 | 36.6 | 63.1 | 53.6 |
| Vasodilator | Isoproterenol hydrochloride | 6.4 | | | |
| | Terbutaline sulfate | | 7.8 | | |
| | Isosorbide mononitrate | | | 13.4 | |
| | Nicotinic acid amide | | | | 8.5 |
| Base | | 11.5 | 11.5 | 19.7 | 14.6 |
| Total (part by mass) | | 54.4 | 55.9 | 96.2 | 76.7 |
| Amount of isoproterenol hydrochloride based on 100 parts by mass of dexmedetomidine hydrochloride (part by mass) | | 17.5 | - | - | - |

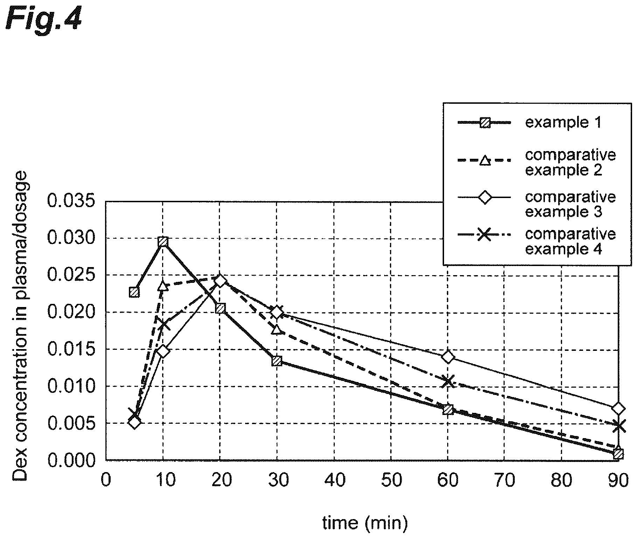

Each microneedle was applied to a male dog, and the increase rate of dexmedetomidine concentration in plasma was analyzed. The results are shown in Figure 4. When a microneedle device comprising isoproterenol hydrochloride was applied (Example 1), the increase of the dexmedetomidine concentration was faster than in the cases where microneedle devices comprising a vasodilator other than isoproterenol hydrochloride and not comprising isoproterenol hydrochloride were applied (Comparative Examples 2 to 4). The amounts of dexmedetomidine hydrochloride and vasodilators which was transferred into the skin by 10 seconds of application are shown in Table 3.

**[Table 3]**

| Component | Amount transferred into skin (µg) | | | |
|---|---|---|---|---|
| | Example 1 | Comparative Example 2 | Comparative Example 3 | Comparative Example 4 |
| Dexmedetomidine hydrochloride | 34.1 | 32.8 | 54.1 | 43.9 |
| Isoproterenol hydrochloride | 6.0 | | | |
| Terbutaline sulfate | | 7.0 | | |
| Isosorbide mononitrate | | | 11.5 | |
| Nicotinic acid amide | | | | 7.0 |

### (Test Example 3)

Microneedle devices having a coating comprising components shown in Table 4 were prepared. A microneedle array used was the same as the one in the Test Example 1.

**[Table 4]**

| Component | | Example 2 | Example 3 | Example 4 | Example 5 |
|---|---|---|---|---|---|
| Dexmedetomidine hydrochloride | | 56.4 | 58.5 | 53.9 | 61.6 |
| Vasodilator | Isoproterenol hydrochloride | 3.9 | 2.0 | 0.6 | 0.2 |
| | Nicotinic acid amide | 10.5 | 10.6 | 9.8 | 11.2 |
| Base | | 11.1 | 11.3 | 11.1 | 12.6 |
| Total (part by mass) | | 81.9 | 82.4 | 75.4 | 85.6 |
| Amount of isoproterenol hydrochloride based on 100 parts by mass of dexmedetomidine hydrochloride (part by mass) | | 6.9 | 3.4 | 1.1 | 0.3 |

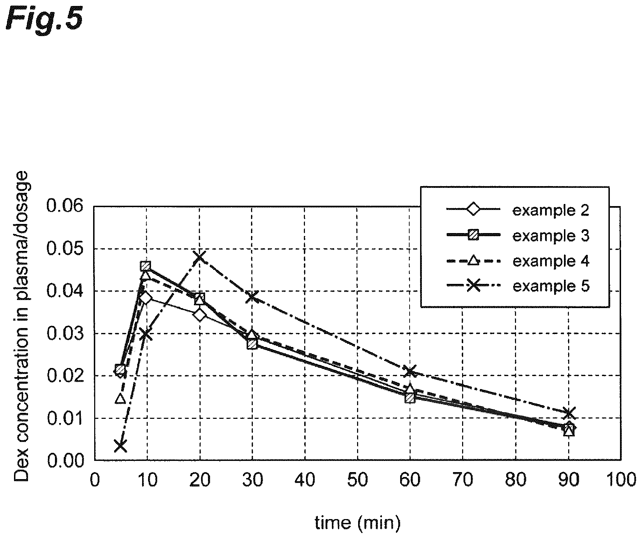

Each microneedle was applied to a male dog, and the increase rate of dexmedetomidine concentration in plasma was analyzed. The results are shown in Figure 5. When the amount of isoproterenol hydrochloride blended was 0.6 parts by mass or more (Examples 2 to 4), the increase of the dexmedetomidine concentration was faster than in the case where the amount of isoproterenol hydrochloride blended was less than 0.6 parts by mass (Example 5). The amounts of dexmedetomidine hydrochloride and isoproterenol hydrochloride which were transferred into the skin by 10 seconds of application are shown in Table 5.

**[Table 5]**

| Component | Amount transferred into skin (µg) | | | |
|---|---|---|---|---|
| | Example 2 | Example 3 | Example 4 | Example 5 |
| Dexmedetomidine hydrochloride | 49.1 | 52.1 | 46.9 | 51.2 |
| Isoproterenol hydrochloride | 3.0 | 1.8 | 0.5 | 0.17 |

### (Test Example 4)

Microneedle devices having a coating comprising components shown in Table 6 were prepared. A microneedle array used was the same as the one in the Test Example 1. After storage at 50°C for one week, the stability of dexmedetomidine hydrochloride in the coating was analyzed by the method described above. Further, the stability of isoproterenol hydrochloride in Examples 7 and 8 was also analyzed.

**[Table 6]**

| | | | Example 6 | Example 7 | Example 8 |
|---|---|---|---|---|---|
| Component | Dexmedetomidine hydrochloride | | 52.6 | 48.1 | 51.8 |
| | Vasodilator | Isoproterenol hydrochloride | 4.4 | 4.0 | 4.0 |
| | | Nicotinic acid amide | 8.6 | 7.8 | 8.4 |
| | Base | | 14.7 | 13.4 | 14.5 |
| | Stabilizer | Sodium pyrosulfite | 1.2 | 0 | 0 |
| | | L-cysteine | 0 | 3.4 | 0 |
| | Total (part by mass) | | 81.5 | 76.7 | 78.7 |
| Result | Amount of dexmedetomidine hydrochloride after storage at 50°C (% based on initial amount) | | 99.7 | 99.4 | 97.9 |
| | Amount of isoproterenol hydrochloride after storage at 50°C (% based on initial amount) | | - | 97.8 | 82.3 |

The results are shown in Table 6. Blending sodium pyrosulfite in the coating resulted in improvement in the stability of dexmedetomidine hydrochloride (Example 6). Blending L-cysteine in the coating resulted in improvement in the stability of dexmedetomidine hydrochloride and isoproterenol hydrochloride (Example 7).

### Reference Signs List

2: Substrate, 4: Microneedle, 6: Coating, 10: Microneedle device

## Claims

1. A microneedle device comprising:
a substrate;
microneedles disposed on the substrate; and
a coating formed on the microneedles,
wherein the coating comprises dexmedetomidine or a pharmaceutically acceptable salt thereof and isoproterenol or a pharmaceutically acceptable salt thereof.

2. The microneedle device according to claim 1, wherein a total amount of isoproterenol and a pharmaceutically acceptable salt thereof is 0.3 parts by mass or more based on 100 parts by mass of a total amount of dexmedetomidine and a pharmaceutically acceptable salt thereof in the coating.

3. The microneedle device according to claim 1 or 2, wherein the coating further comprises a stabilizer.

4. The microneedle device according to claim 1 or 2, wherein the coating further comprises L-cysteine.

5. The microneedle device according to claim 1 or 2, wherein the coating further comprises sodium pyrosulfite.

## Patentansprüche

1. Mikronadelvorrichtung, umfassend:
ein Substrat;
auf dem Substrat angeordnete Mikronadeln und
eine auf den Mikronadeln gebildete Beschichtung,
wobei die Beschichtung Dexmedetomidin oder ein pharmazeutisch verträgliches Salz davon und Isoproterenol oder ein pharmazeutisch verträgliches Salz davon umfasst.

2. Mikronadelvorrichtung gemäß Anspruch 1, wobei eine Gesamtmenge an Isoproterenol und einem pharmazeutisch verträglichen Salz davon 0,3 Massenteile oder mehr bezogen auf 100 Massenteile einer Gesamtmenge an Dexmedetomidin und einem pharmazeutisch verträglichen Salz davon in der Beschichtung beträgt.

3. Mikronadelvorrichtung gemäß Anspruch 1 oder 2, wobei die Beschichtung ferner einen Stabilisator umfasst.

4. Mikronadelvorrichtung gemäß Anspruch 1 oder 2, wobei die Beschichtung ferner L-Cystein umfasst.

5. Mikronadelvorrichtung gemäß Anspruch 1 oder 2, wobei die Beschichtung ferner Natriumpyrosulfit umfasst.

## Revendications

1. Dispositif à micro-aiguilles comprenant :
un substrat ;
des micro-aiguilles disposées sur le substrat ; et
un revêtement formé sur les micro-aiguilles,
le revêtement comprenant de la dexmédétomidine ou son sel pharmaceutiquement acceptable et de l'isoprotérénol ou son sel pharmaceutiquement acceptable.

2. Dispositif à micro-aiguilles selon la revendication 1, une quantité totale d'isoprotérénol et son sel pharmaceutiquement acceptable étant de 0,3 partie en masse ou plus sur la base de 100 parties en masse d'une quantité totale de dexmédétomidine et son sel pharmaceutiquement acceptable dans le revêtement.

3. Dispositif à micro-aiguilles selon la revendication 1 ou 2, le revêtement comprenant en outre un agent stabilisant.

4. Dispositif à micro-aiguilles selon la revendication 1 ou 2, le revêtement comprenant en outre de la L-cystéine.

5. Dispositif à micro-aiguilles selon la revendication 1 ou 2, le revêtement comprenant en outre du pyrosulfite de sodium.
